(19) Europäisches Patentamt / European Patent Office / Office européen des brevets

(11) **EP 4 091 999 B1**

(12) **EUROPEAN PATENT SPECIFICATION**

(45) Date of publication and mention
of the grant of the patent:
**08.04.2026   Bulletin 2026/15**

(21) Application number: **21175364.5**

(22) Date of filing: **21.05.2021**

(51) International Patent Classification (IPC):
$C03C\ 3/091^{(2006.01)}$    $A61L\ 2/10^{(2026.01)}$
$C02F\ 1/32^{(2023.01)}$    $C03B\ 5/027^{(2006.01)}$
$C03C\ 4/08^{(2006.01)}$    $F24F\ 8/22^{(2021.01)}$

(52) Cooperative Patent Classification (CPC):
**C03C 3/091; A61L 2/10; A61L 2/26; C03B 5/185;
C03C 4/085**

(54) **GLASS HAVING HIGH UV TRANSMITTANCE AND HIGH SOLARIZATION RESISTANCE**

GLAS MIT HOHER UV-DURCHLÄSSIGKEIT UND HOHER SOLARISATIONSBESTÄNDIGKEIT

VERRE POSSÉDANT UN FACTEUR DE TRANSMISSION UV ET UNE RÉSISTANCE À LA SOLARISATION ÉLEVÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**23.11.2022   Bulletin 2022/47**

(73) Proprietor: **SCHOTT AG
55122 Mainz (DE)**

(72) Inventors:
• **Grimm, Malte
95666 Mitterteich (DE)**
• **Eichholz, Rainer
60323 Frankfurt am Main (DE)**
• **Rasp, Josef
95652 Waldsassen (DE)**

(74) Representative: **Fuchs Patentanwälte
Partnerschaft mbB
Tower 185
Friedrich-Ebert-Anlage 35-37
60327 Frankfurt am Main (DE)**

(56) References cited:
WO-A1-2018/204549    US-A- 3 381 078
US-A- 4 557 743    US-A- 5 045 509
US-A- 5 610 108    US-A1- 2002 121 114

**Description**

The present invention relates to a glass having high UV transmittance and high solarization resistance.

**Background of the invention**

Short-wavelength ultraviolet (UVC) light is used to kill or inactivate microorganisms by destroying nucleic acids and disrupting their DNA, leaving them unable to perform vital cellular functions. This is used in a variety of applications, such as purification of food, air, and/or water. A wavelength of 260 nm is particularly relevant in this respect as nucleic acids such as DNA have an absorption maximum at this wavelength. For example, the A260/A280 ratio is often used to indicate the purity of a DNA sample. "A260" refers to the absorption at a wavelength of 260 nm and "A280" refers to the absorption at a wavelength of 280 nm. A high A260/A280 ratio indicates a high purity of the DNA sample. The reason is that DNA strongly absorbs at 260 nm whereas protein contaminations mainly absorb at 280 nm. Thus, a high A260/A280 ratio means that the sample contains a high amount of DNA and a low amount of protein.

There are two main demands on glasses used in such lamps. First, the glasses should have a high UV transmittance, in particular in the UVC region. Second, the glasses should have a high solarization resistance so that their performance does not decrease too quickly upon use. Thus, a high solarization resistance increases the lifetime of the UV lamps. Consequently, there is a need for glasses combining both a high UV transmittance and a high solarization resistance.

Moreover, in view of the climate change, reducing consumption of fossil energy is more and more desired throughout different industries. In particular, the energy consumption during production of glasses is high. In order to improve the climate footprint of glass production, electrical energy can be used. Electrical energy may for example be produced from renewable energy sources such as wind energy or solar energy.

Using electrical energy, an electric current may be passed through the bath of molten glass between electrodes immersed in the molten glass. However, the choice of materials of the electrodes may be critical, in particular when glasses having high UV transmittance and high solarization resistance are concerned.

US 2002/0121114 A1 discloses a meltdown device for the production of high-UV transmittive glass types, comprising a meltdown tank for a melt bath; a feed opening for the supplying or laying-in of highly pure raw material for the melt bath; a draw-off opening for the drawing-off of material melted in the melt tank; a cover arranged above the melt tank, in which the feed opening to the melt tank is arranged above the melt bath in the region of the cover; the draw-off opening is arranged in the zone of the bottom of the melt tank; and a heating arrangement characterized in that the heating arrangement comprises heating elements, in particular electrodes that are arranged on the melt tank in the zone of the melt bath, as well as an agitating arrangement for the stirring of the melt bath and uniform intermixing and sub-mixing into the melt of material from the mixture lying on the melt surface.

It is an object of the present invention to overcome the disadvantages described above with respect to the state of the art by providing means which allow a reduction of fossil energy consumption during production of glasses having high UV transmittance and high solarization resistance. It is further the object of the present invention to provide such glass with particularly high quality.

**Description of the invention**

For obtaining glass articles having high UV transmittance and high solarization resistance, so far technologies associated with high consumption of fossil energy had to be used.

However, the present invention provides a glass having high UV transmittance and high solarization resistance that can be obtained with reduced consumption of fossil energy without substantially compromising UV transmittance and solarization resistance. The invention is based on the finding that molybdenum electrodes can be used during production and that a certain amount of molybdenum emanating from the electrodes into the melt can be tolerated if reducing melting conditions are applied.

In one aspect, the invention relates to a glass, wherein the amount of $MoO_3$ is from 0.1 ppm to 30.0 ppm, and wherein molybdenum is present in such oxidation states that the transmittance at a wavelength of 260 nm is at least 65% at a reference thickness of 1.0 mm, wherein the glass comprises the following components in the indicated amounts (in wt.-%):

| Component | Amount (wt.-%) |
|---|---|
| $SiO_2$ | 60.0-80.0 |
| $B_2O_3$ | 0.5-30.0 |

(continued)

| Component | Amount (wt.-%) |
|---|---|
| $Al_2O_3$ | 0.5-10.0 |
| $Na_2O$ | 2.0-20.0 |
| $K_2O$ | 0-20.0 |
| BaO | 0-15.0 |

The present disclosure refers to molybdenum present in the glasses as "$MoO_3$". However, that is not supposed to mean that molybdenum is present in the glass predominantly as molybdenum (VI). Rather, the term "$MoO_3$" simply follows the common way of referring to molybdenum in the glass and refers to the total amount of molybdenum irrespective of the oxidation state.

The present invention includes reducing melting conditions. Thus, in one aspect of the invention the amount of molybdenum (VI) in the glasses is rather low. Molybdenum (VI) is preferably not the predominant molybdenum species in the glass. In the glasses of the invention, molybdenum is present in such oxidation states that the transmittance at a wavelength of 260 nm is at least 65% (at a reference thickness of 1.0 mm).

In one aspect, the invention relates to a glass comprising the following components in the indicated amounts (in wt.-%):

| Component | Amount (wt.-%) |
|---|---|
| $SiO_2$ | 60.0-80.0 |
| $B_2O_3$ | 0.5-30.0 |
| $Al_2O_3$ | 0.5-10.0 |
| $Na_2O$ | 2.0-20.0 |
| $K_2O$ | 0-20.0 |
| BaO | 0-15.0 |

In one aspect of the invention, the partial pressure of oxygen ($pO_2$) in the glass melt at a temperature of 1500°C is 0.5 bar or less when the glass melt is produced from the glass by inductively heating to a temperature of 1500°C in a platinum crucible under argon atmosphere. The $pO_2$ at 1500°C may for example be at most 0.4 bar, at most 0.3 bar, or at most 0.2 bar. In some embodiments, the $pO_2$ may for example be at least 0.01 bar, at least 0.02 bar, at least 0.05 bar, or at least 0.1 bar. The $pO_2$ may for example be from 0.01 bar to 0.5 bar, from 0.02 bar to 0.4 bar, from 0.05 bar to 0.3 bar, or from 0.1 bar to 0.2 bar. A comparably low $pO_2$ is advantageous as is associated with lower oxidation states of molybdenum. However, the $pO_2$ should not be too low in order to avoid occurrence of metallic molybdenum.

The $pO_2$ of the glass melt may for example be determined based on the voltage between a reference electrode and a measuring electrode both of which are positioned in the glass melt. The $pO_2$ can be calculated from the voltage between the electrodes by using the Nernst equation. A platinum plate can be used as measuring electrode. The reference electrode may comprise a platinum wire positioned inside a $ZrO_2$ ceramic tube that is closed at the tip, wherein the platinum wire is in electrically conducting contact with the wall of the $ZrO_2$ tube. The $ZrO_2$ ceramic may be yttrium-stabilized, calcium-stabilized, or magnesium-stabilized (see for example EP 1 101 740 A1, par. [0012], [0013]). For the measurement of the $pO_2$ of the glass melt, pure oxygen is flowing around the platinum wire so that there is a constant $pO_2$ of 1.0 bar at the platinum wire inside the reference electrode. The $ZrO_2$ is an oxygen conductor and forms a bridge between the platinum inside the reference electrode and the glass melt, and thus indirectly also with the platinum measuring electrode inside the glass melt. Oxygen ions migrate. The concentration cell "platinum ($pO_2$=constant=1.0 bar) / $ZrO_2$ / glass melt / platinum ($pO_2$ of the glass melt)" generates a voltage. The voltage between the reference electrode and the measuring electrode is proportional to the $pO_2$ in the glass melt and can thus be converted to determine the $pO_2$ in the glass melt based on the Nernst equation.

The glass may comprise the following components in the indicated amounts (in wt.-%):

| Component | Amount (wt.-%) |
|---|---|
| $SiO_2$ | 60.0-75.0 |

(continued)

| Component | Amount (wt.-%) |
|---|---|
| $B_2O_3$ | 0.5-15.0 |
| $Al_2O_3$ | 0.5-7.5 |
| $Na_2O$ | 2.5-15.0 |
| $K_2O$ | 2.0-16.0 |
| BaO | 2.5-12.5 |

The glass may comprise the following components in the indicated amounts (in wt.-%):

| Component | Amount (wt.-%) |
|---|---|
| $SiO_2$ | 60.0-75.0 |
| $B_2O_3$ | 0.5-5.0 |
| $Al_2O_3$ | 0.5-5.0 |
| $Na_2O$ | 7.0-10.0 |
| $K_2O$ | 8.0-12.0 |
| BaO | 4.5-10.0 |

Preferably, the glass of the invention has a transmittance at a wavelength of 260 nm of at least 65.0%, for example at least 66.0%, at least 67.0%, at least 68.0%, at least 69.0%, at least 70.0%, more preferably at least 71.0%, more preferably at least 72.0%, more preferably at least 73.0%, more preferably at least 74.0%, more preferably at least 75.0%, more preferably at least 76.0%, more preferably at least 77.0%, more preferably at least 78.0%, more preferably at least 79.0%, more preferably at least 80.0%. Transmittance values in the present disclosure refer to a reference thickness of 1.0 mm of the glass if not indicated otherwise. That does not mean that the glass necessarily has to have a thickness of 1.0 mm. Rather, the reference thickness simply indicates what the transmittance would be if the glass had a thickness of 1.0 mm. The transmittance at a reference thickness may be determined by measuring the transmittance of a sample having a thickness of 1.0 mm. Alternatively, the transmittance at a thickness of 1.0 mm may also be determined by measuring the transmittance at another sample thickness, for example at a sample thickness of 0.7 mm, and then extrapolating what the transmittance would be at a thickness of 1.0 mm. Generally speaking, the extrapolation of a transmittance T1 at a thickness d1 to a transmittance T2 at a thickness d2 can be done using the following formula:
T2 = ((T1/P)^(d2/d1))*P, wherein P is the wavelength-dependent reflection coefficient (P = P($\lambda$)) given in the unit "%". P can be determined via the Sellmeier n coefficient.

In embodiments of the invention, the transmittance at a wavelength of 260 nm may be at most 99.0%, at most 97.5%, at most 95.0%, at most 94.0%, at most 93.0%, at most 92.0%, at most 91.0%, at most 90.0%, at most 89.0%, at most 88.0%, at most 87.0%, at most 86.0%, at most 85.0%, at most 84.0%, at most 83.0%, at most 82.0% (at a reference thickness of 1.0 mm). The transmittance at a wavelength of 260 nm may for example be in a range of from 65.0% to 99.0%, from 66.0% to 97.5%, from 67.0% to 95.0%, from 68.0% to 94.0%, from 69.0% to 93.0%, from 70.0% to 92.0%, from 71.0% to 91.0%, from 72.0% to 90.0%, from 73.0% to 89.0%, from 74.0% to 88.0%, from 75.0% to 87.0%, from 76.0% to 86.0%, from 77.0% to 85.0%, from 78.0% to 84.0%, from 79.0% to 83.0%, or from 80.0% to 82.0%.

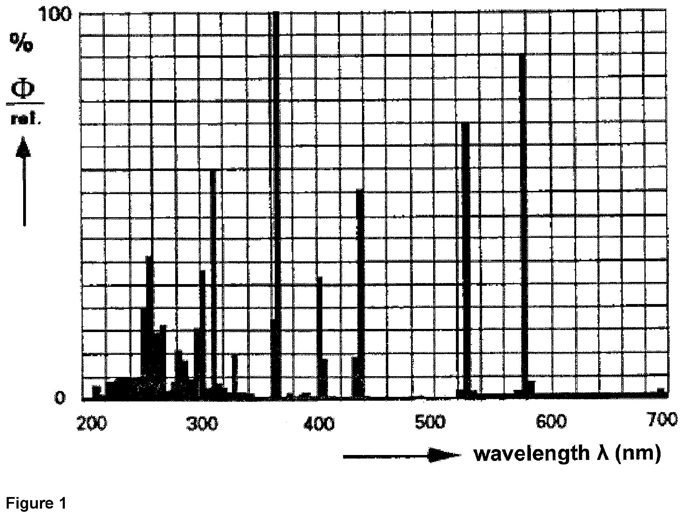

The glasses of the invention are characterized by a particularly high solarization resistance. The solarization resistance can be determined by irradiating the glass with a HOK 4 lamp for 144 hours and comparing the transmittance (at a reference thickness of 1.0 mm) at a wavelength of 260 nm before and after irradiation. The term "HOK 4 lamp" refers to the high pressure mercury-vapor lamp HOK 4/120 of Phillips. The emission spectrum of the HOK 4 lamp is shown in Figure 1. The main emission of the lamp is at a wavelength of 365 nm. The power density at 200-280 nm in a distance of 1 m is 850 $\mu$W/cm$^2$. For the irradiation for 144 hours of the present invention, the distance between the HOK 4 lamp and the sample is chosen to be 7 cm.

The lower the difference between the transmittance before and after irradiation is, the higher is the solarization resistance. For example, there may be two glasses, each having a transmittance (at a reference thickness of 1.0 mm) of 80% at a wavelength of 260 nm (before irradiation). After irradiation with the HOK 4 lamp for 144 hours, the transmittance may be 75% for the first glass and 70% for the second glass. Thus, the difference between transmittance (at a reference thickness of 1.0 mm) at a wavelength of 260 nm prior to irradiation with the HOK 4 lamp and transmittance (at a reference thickness of 1.0 mm) at a wavelength of 260 nm after irradiation with the HOK 4 lamp for 144 hours is 5% for the first glass and 10% for the second glass. Hence, the solarization resistance of the first glass is higher as compared to the second glass because the difference between the transmittance before and after irradiation is lower for the first glass as compared to the second glass. A high solarization resistance is associated with low solarization and *vice versa*. A high solarization correlates with a high induced extinction Ex-t$_{ind}$.

The induced extinction Ext$_{ind}$ can be determined based on the transmittance before and after irradiation with the HOK 4 lamp for 144 hours and the thickness of the glass sample using the following formula:

$$Ext_{ind} = -\frac{ln\frac{T_{after}}{T_{before}}}{d}$$

Ext$_{ind}$ is the induced extinction, T$_{after}$ is the transmittance after irradiation with the HOK 4 lamp for 144 hours, T$_{before}$ is the transmittance before irradiation with the HOK 4 lamp for 144 hours, d is the sample thickness, and ln is the natural logarithm. If not indicated otherwise, the sample thickness d is given in cm so that the induced extinction is given in 1/cm. If not indicated otherwise, the transmittance before and after irradiation with the HOK 4 lamp are given for a wavelength of 260 nm. Thus, the induced extinction as described in the present disclosure refers to the induced extinction at a wavelength of 260 nm if not indicated otherwise.

In one aspect, the induced extinction at a wavelength of 260 nm is at most 3.5/cm, at most 3.2/cm, at most 3.0/cm, at most 2.9/cm, at most 2.8/cm, at most 2.7/cm, at most 2.6/cm, at most 2.5/cm, at most 2.4/cm, at most 2.3/cm, at most 2.2/cm, at most 2.1/cm, at most 2.0/cm, at most 1.9/cm, at most 1.8/cm, at most 1.7/cm, at most 1.6/cm, at most 1.5/cm, at most 1.4/cm, or at most 1.3/cm. The induced extinction at a wavelength of 260 nm may for example be at least 0.01/cm, at least 0.02/cm, at least 0.05/cm, at least 0.1/cm, at least 0.2/cm, at least 0.3/cm, at least 0.4/cm, at least 0.5/cm, at least 0.55/cm, at least 0.6/cm, at least 0.65/cm, at least 0.7/cm, at least 0.75/cm, at least 0.8/cm, at least 0.85/cm, at least 0.9/cm, at least 0.95/cm, at least 1.0/cm, at least 1.05/cm, or at least 1.1/cm. The induced extinction at a wavelength of 260 nm may for example be from 0.01/cm to 3.5/cm, from 0.02/cm to 3.2/cm, from 0.05/cm to 3.0/cm, from 0.1/cm to 2.0/cm, from 0.2/cm to 2.8/cm, from 0.3/cm to 2.7/cm, from 0.4/cm to 2.6/cm, from 0.5/cm to 2.5/cm, from 0.55/cm to 2.4/cm, from 0.6/cm to 2.3/cm, from 0.65/cm to 2.2/cm, from 0.7/cm to 2.1/cm, from 0.75/cm to 2.0/cm, from 0.8/cm to 1.9/cm, from 0.85/cm to 1.8/cm, from 0.9/cm to 1.7/cm, from 0.95/cm to 1.6/cm, from 1.0/cm to 1.5/cm, from 1.05/cm to 1.4/cm, or from 1.1/cm to 1.3/cm.

In one aspect, the glass of the invention is characterized by a high UV transmittance and a low induced extinction. This combination is associated with a high transmittance (at a reference thickness of 1.0 mm) at a wavelength of 260 nm after irradiation with the HOK 4 lamp for 144 hours. The transmittance (at a reference thickness of 1.0 mm) at a wavelength of 260 nm after irradiation with the HOK 4 lamp for 144 hours may for example be at least 54.0%, at least 55.0%, more preferably at least 56.0%, more preferably at least 57.0%, more preferably at least 58.0%, more preferably at least 59.0%, more preferably at least 60.0%, more preferably at least 61.0%, more preferably at least 62.0%, more preferably at least 63.0%, more preferably at least 64.0%, more preferably at least 65.0%, more preferably at least 66.0%, more preferably at least 67.0%, more preferably at least 68.0%, more preferably at least 69.0%, more preferably at least 70.0%, more preferably at least 71.0%, more preferably at least 72.0%, more preferably at least 73.0%, more preferably at least 74.0%.

The transmittance (at a reference thickness of 1.0 mm) at a wavelength of 260 nm after irradiation with the HOK 4 lamp for 144 hours may for example be at most 95.0%, at most 94.0%, at most 93.0%, at most 92.0%, at most 91.0%, at most 90.0%, at most 89.0%, at most 88.0%, at most 87.0%, at most 86.0%, at most 85.0%, at most 84.0%, at most 83.0%, at most 82.0%, at most 81.0%, at most 80.0%, at most 79.0%, at most 78.0%, at most 77.0%, at most 76.0%, or at most 75.0%. The transmittance at a wavelength of 260 nm may for example be in a range of from 54.0% to 95.0%, from 55.0% to 94.0%, from 56.0% to 93.0%, from 57.0% to 92.0%, from 58.0% to 91.0%, from 59.0% to 90.0%, from 60.0% to 89.0%, from 61.0% to 88.0%, from 62.0% to 87.0%, from 63.0% to 86.0%, from 64.0% to 85.0%, from 65.0% to 84.0%, from 66.0% to 83.0%, from 67.0% to 82.0% from 68.0% to 81.0%, from 69.0% to 80.0%, from 70.0% to 79.0%, from 71.0% to 78.0%, from 72.0% to 77.0%, from 73.0% to 76.0%, or from 74.0% to 75.0%.

Molybdenum (Mo) electrodes are subject to corrosion and removal of electrode material during use. Molybdenum emanating from the electrodes can then also be found in the molten glass material. This leads to contaminations of the final glass articles produced from the molten glass. Such contaminations are in turn associated with compromised properties of the resulting glasses, in particular with reduced UV transmittance and/or reduced solarization resistance. The present invention includes the finding that molybdenum electrodes can be used during production and that a certain amount of molybdenum emanating from the electrodes into the melt can be tolerated if reducing melting conditions are applied.

The glass of the invention comprises MoO$_3$ in an amount of from 0.1 ppm to 30.0 ppm. The amount of MoO$_3$ may for example be at least 0.2 ppm, at least 0.4 ppm, at least 0.5 ppm, at least 1.0 ppm, at least 2.0 ppm, at least 3.0 ppm, or at least 5.0 ppm. The amount of $MoO_3$ may for example be at most 25.0 ppm, at most 22.5 ppm, at most 20.0 ppm, at most 17.5 ppm, at most 15.0 ppm, at most 12.5 ppm, or at most 10.0 ppm. The amount of $MoO_3$ may for example be from 0.2 ppm to 25.0 ppm, from 0.4 ppm to 22.5 ppm, from 0.5 ppm to 20.0 ppm, from 1.0 ppm to 17.5 ppm, from 2.0 ppm to 15.0 ppm, from 3.0 ppm to 12.5 ppm, or from 5.0 ppm to 10.0 ppm.

The glass of the invention comprises $SiO_2$ in an amount of from 60.0 to 80.0 wt.-%, preferably from 60.0 to 75.0 wt.-% or from 62.5 to 75.0 wt.-%. The amount of $SiO_2$ is at least 60.0%, for example at least 62.5%. The amount of $SiO_2$ may for example be at most 80.0 wt.-% or at most 75.0 wt.-%.

The glass of the invention comprises $B_2O_3$ in an amount of from 0.5 to 30.0 wt.-% preferably from 0.5 to 25.0 wt.-%, from 0.5 to 20.0 wt.-%, from 0.5 to 20.0 wt.-%, from 0.5 to 15.0 wt.-%, from 0.5 to 10.0 wt.-%, from 0.5 to 5.0 wt.-% or from 1.0 to 4.0 wt.-%. The amount of $B_2O_3$ may for example be at least 0.5 wt.-% or at least 1.0 wt.-%. The amount of $B_2O_3$ may for example be at most 30.0 wt.-%, at most 25.0 wt.-%, at most 20.0 wt.-%, at most 15.0 wt.-%, at most 10.0 wt.-%, at most 5.0 wt.-% or at most 4.0 wt.-%.

The glass of the invention comprises $Al_2O_3$ in an amount of from 0.5 to 10.0 wt.-% preferably from 0.5 to 7.5 wt.-%, from 0.5 to 5.0 wt.-% or from 1.0 wt.-% to 4.5 wt.-%. The amount of $Al_2O_3$ may for example be at least 0.5 wt.-% or at least 1.0 wt.-%. The amount of $Al_2O_3$ may for example be at most 10.0 wt.-%, at most 7.5 wt.-%, at most 5.0 wt.-% or at most 4.5 wt.-%.

The glass of the invention comprises $Na_2O$ in an amount of from 2.0 to 20.0 wt.-% from 2.25 to 19.0 wt.-%, or from 2.5 to 18.0 wt.-%, preferably from 5.0 wt.-% to 17.5 wt.-%, from 6.0 wt.-% to 15.0 wt.-% or from 7.0 to 10.0 wt.-%. The amount of $Na_2O$ may for example be at least 2.0 wt.-%, at least 2.25 wt.-%, at least 2.5 wt.-%, at least 5.0 wt.-%, at least 6.0 wt.-% or at least 7.0 wt.-%. The amount of $Na_2O$ may for example be at most 20.0 wt.-%, at most 19.0 wt.-%, at most 18.0 wt.-%, at most 17.5 wt.-%, at most 15.0 wt.-% or at most 10.0 wt.-%.

The glass of the invention may comprise $K_2O$ in an amount of from 0 to 20.0 wt.-% for example from 1.0 to 18.0 wt.-%, from 2.0 to 16.0 wt.-%, from 5.0 to 14.0 wt.-% or from 8.0 to 12.0 wt.-%. The amount of $K_2O$ may for example be at least 1.0 wt.-%, at least 2.0 wt.-%, at least 5.0 wt.-% or at least 8.0 wt.-%. The amount of $K_2O$ may for example be at most 20.0 wt.-%, at most 18.0 wt.-%, at most 16.0 wt.-%, at most 14.0 wt.-% or at most 12.0 wt.-%. In some embodiments, the amount of $K_2O$ is at most 1.0 wt.-%, at most 0.5 wt.-%, at most 0.2 wt.-% or at most 0.1 wt.-%. The glass of the invention may comprise $Li_2O$. The amount of $Li_2O$ may for example be in an amount of from 0 to 5.0 wt.-%, for example from 0.1 to 2.0 wt.-% or from 0.5 to 1.5 wt.-%. The amount of $Li_2O$ may for example be at least 0.1 wt.-%, at least 0.2 wt.-% or at least 0.5 wt.-%. The amount of $Li_2O$ may for example be at most 5.0 wt.-%, at most 2.0 wt.-% or at most 1.5 wt.-%. In some embodiments, the amount of $Li_2O$ is at most 1.0 wt.-%, at most 0.5 wt.-%, at most 0.2 wt.-% or at most 0.1 wt.-%.

Preferably, the sum of the amounts of $Li_2O$, $Na_2O$ and $K_2O$ is in a range of from 4.0 wt.-% to 30.0 wt.-% or from 5.0 to 30.0 wt.-%, for example from 7.5 wt.-% to 27.5 wt.-%, from 10.0 wt.-% to 25.0 wt.-%, from 12.5 wt.-% to 22.5 wt.-% or from 15.0 wt.-% to 20.0 wt.-%. The sum of the amounts of $Li_2O$, $Na_2O$ and $K_2O$ may for example be at least 4.0 wt.-%, at least 5.0 wt.-%, at least 7.5 wt.-%, at least 10.0 wt.-%, at least 12.5 wt.-% or at least 15.0 wt.-%. The sum of the amounts of $Li_2O$, $Na_2O$ and $K_2O$ may for example be at most 30.0 wt.-%, at most 27.5 wt.-%, at most 25.0 wt.-%, at most 22.5 wt.-% or at most 20.0 wt.-%.

Preferably, the sum of the amounts of $Na_2O$ and $K_2O$ is in a range of from 3.5 wt.-% to 30.0 wt.-% or from 5.0 to 30.0 wt.-%, for example from 7.5 wt.-% to 27.5 wt.-%, from 10.0 wt.-% to 25.0 wt.-%, from 12.5 wt.-% to 22.5 wt.-% or from 15.0 wt.-% to 20.0 wt.-%. The sum of the amounts of $Na_2O$ and $K_2O$ may for example be at least 3.5 wt.-%, at least 5.0 wt.-%, at least 7.5 wt.-%, at least 10.0 wt.-%, at least 12.5 wt.-% or at least 15.0 wt.-%. The sum of the amounts of $Na_2O$ and $K_2O$ may for example be at most 30.0 wt.-%, at most 27.5 wt.-%, at most 25.0 wt.-%, at most 22.5 wt.-% or at most 20.0 wt.-%.

The glasses of the invention may comprise $BaO$ in an amount of from 0 to 15.0 wt.-%, preferably from 2.5 to 12.5 wt.-%, from 4.5 to 10.0 wt.-% or from 5.0 to 8.0 wt.-%. The amount of $BaO$ may for example be at least 2.5 wt.-%, at least 4.5 wt.-% or at least 5.0 wt.-%. The amount of $BaO$ may for example be at most 15.0 wt.-%, at most 12.5 wt.-%, at most 10.0 wt.-% or at most 8.0 wt.-%. In some embodiments, the amount of $BaO$ is at most 1.0 wt.-%, at most 0.5 wt.-%, at most 0.2 wt.-% or at most 0.1 wt.-%.

The glass may consist of $SiO_2$, $B_2O_3$, $Al_2O_3$, $Na_2O$, $K_2O$ and $BaO$ to an extent of at least 95.0 wt.-%, more preferably at least 97.0 wt.-%, most preferably at least 99.0 wt.-%.

Preferably, the amount of $Fe_2O_3$ is less than 100 ppm, more preferably less than 50 ppm, more preferably less than 20 ppm, more preferably less than 15 ppm, more preferably less than 10 ppm.

Preferably, the amount of $TiO_2$ is less than 100 ppm, more preferably less than 50 ppm, more preferably less than 20 ppm, more preferably less than 15 ppm, more preferably less than 10 ppm.

Preferably, the amount of $NiO$ is less than 100 ppm, more preferably less than 50 ppm, more preferably less than 20 ppm, more preferably less than 15 ppm, more preferably less than 10 ppm.

Preferably, the amount of $Cr_2O_3$ is less than 100 ppm, more preferably less than 50 ppm, more preferably less than 20 ppm, more preferably less than 15 ppm, more preferably less than 10 ppm, more preferably less than 5 ppm.

Preferably, the amount of at least one, at least two, or at least three of $Fe_2O_3$, $TiO_2$, $NiO$ and $Cr_2O_3$ is less than 100 ppm, more preferably less than 50 ppm, more preferably less than 20 ppm, more preferably less than 15 ppm, more preferably less than 10 ppm.

Preferably, the amount of $Fe_2O_3$, $TiO_2$, NiO and $Cr_2O_3$ is less than 100 ppm, more preferably less than 50 ppm, more preferably less than 20 ppm, more preferably less than 15 ppm, more preferably less than 10 ppm.

The term "ppm" as used herein refers to ppm on a weight basis if not indicated otherwise. For example, 1000 ppm correspond to 0.1 wt.-%.

Preferably, the amount of $ZrO_2$ is less than 1.0 wt.-%, less than 0.5 wt.-%, less than 0.2 wt.-% or less than 0.1 wt.-%.

The glass of the invention may comprise CI in an amount of more than 100 ppm, more than 200 ppm or more than 500 ppm, for example at least 0.1 wt.-%. Preferably, the amount of CI is at most 1.5 wt.-%, at most 1.2 wt.-%, at most 1.0 wt.-% or at most 0.8 wt.-%. The amount of CI may for example be from 100 ppm to 1.5 wt.-%, from 200 ppm to 1.2 wt.-%, from 500 ppm to 1.0 wt.-% or from 0.1 wt.-% to 0.8 wt.-%.

In one aspect of the present invention, the glass has a composition that makes it particularly resistant against the influence of $MoO_3$ on UV transmittance and/or solarization. The glass preferably has a good UV transmittance and a good solarization resistance even though certain amounts of $MoO_3$ are present in the glass. A certain amount of $MoO_3$ can be accepted in the glasses of the invention, in particular due to the reducing melting conditions applied.

Increasing amounts of $MoO_3$ are associated with decreasing transmittance at a wavelength of 260 nm. However, in one aspect of the present invention the glass composition is such that molybdenum induced extinction is low. Therefore, higher amounts of $MoO_3$ then expected may be acceptable in the glasses of the present invention.

The molybdenum induced extinction $MoExt_{ind}$ can be determined based on the transmittance before and after irradiation with the HOK 4 lamp for 144 hours, the thickness of the glass sample, and the amount of $MoO_3$ using the following formula:

$$MoExt_{ind} = -\frac{ln\frac{T_{after}}{T_{before}}}{d \cdot Mo}$$

$MoExt_{ind}$ is the molybdenum induced extinction, $T_{after}$ is the transmittance after irradiation with the HOK 4 lamp for 144 hours, $T_{before}$ is the transmittance before irradiation with the HOK 4 lamp for 144 hours, d is the sample thickness, ln is the natural logarithm, and Mo is the amount of $MoO_3$ in ppm. If not indicated otherwise, the sample thickness d is given in cm so that the molybdenum induced extinction is given in 1/(cm*ppm). If not indicated otherwise, the transmittance before and after irradiation with the HOK 4 lamp is given for a wavelength of 260 nm. Thus, the molybdenum induced extinction as described in the present disclosure refers to the molybdenum induced extinction at a wavelength of 260 nm if not indicated otherwise.

In one aspect, the molybdenum induced extinction at a wavelength of 260 nm is at most 0.20/(cm*ppm), at most 0.15/(cm*ppm), at most 0.14/(cm*ppm), at most 0.13/(cm*ppm), at most 0.12/(cm*ppm), at most 0.11/(cm*ppm), or at most 0.10/(cm*ppm) at a reference amount of $MoO_3$ of 20 ppm. The molybdenum induced extinction at a wavelength of 260 nm may for example be at least 0.01/(cm*ppm), at least 0.02/(cm*ppm), at least 0.03/(cm*ppm), at least 0.04/(cm*ppm), at least 0.05/(cm*ppm), at least 0.06/(cm*ppm), or at least 0.07/(cm*ppm) at a reference amount of $MoO_3$ of 20 ppm. The molybdenum induced extinction at a wavelength of 260 nm may for example be from 0.01/(cm*ppm) to 0.20/(cm*ppm), from 0.02/(cm*ppm) to 0.15/(cm*ppm), from 0.03/(cm*ppm) to 0.14/(cm*ppm), from 0.04/(cm*ppm) to 0.13/(cm*ppm), from 0.05/(cm*ppm) to 0.12/(cm*ppm), from 0.06/(cm*ppm) to 0.11/(cm*ppm), or from 0.07/(cm*ppm) to 0.10/(cm*ppm) at a reference amount of $MoO_3$ of 20 ppm.

The invention also relates to a method of producing a glass of the invention, the method comprising the following steps:

a) Melting glass raw materials under reducing melting conditions, the melting step comprising the use of molybdenum electrodes, wherein reducing melting conditions comprises adding one or more reducing agents, for example sugar, to the glass raw materials,

b) Optionally refining the melt,

c) Cooling the melt,

wherein the method comprises temperatures of 1440 °C or more, wherein the method involves viscosities of 2.5 dPas or less.

The method is characterized by reducing melting conditions such that molybdenum is present in such oxidation states that the transmittance at a wavelength of 260 nm is at least 65% (at a reference thickness of 1.0 mm). Such conditions result in the compromising influence of molybdenum on UV transmittance and solarization resistance to be drastically reduced so that a certain amount of molybdenum emanating from the electrodes into the melt can be tolerated without substantially compromising UV transmittance and solarization resistance. The reducing character of the melt is also influenced by the temperature. The method comprises temperatures of 1440°C or more, for example 1500°C or more. The viscosity at a given temperature is dependent on the glass composition. The method of the invention involves viscosities of 2.5 dPas or less.

However, the conditions should not be too reducing in order to avoid metallic molybdenum.

Providing reducing melting conditions preferably comprises adding one or more reducing agent, for example one or more carbohydrate, in particular one or more sugar, for example sucrose, to the glass raw materials, in particular in an amount of from 0.1 to 1.0 wt.-%, for example from 0.2 to 0.6 wt.-%. Glass raw materials comprising nitrates are preferably avoided. Preferably, the amount of nitrates is at most 200 ppm, at most 100 ppm, at most 50 ppm, at most 20 ppm, at most 10 ppm, at most 5 ppm, at most 2 ppm, or at most 1 ppm.

The optional refining step preferably comprises using Cl as refining agent. Cl is a preferred refining agent as it has a reducing character as well. Oxidizing refining agents are preferably avoided. Preferably, the amount of oxidizing refining agents is at most 200 ppm, at most 100 ppm, at most 50 ppm, at most 20 ppm, at most 10 ppm, at most 5 ppm, at most 2 ppm, or at most 1 ppm.

The invention also relates to the use of glass of the invention for or as UVC detectors, envelope in germicidal lamps, UV-LED (in particular UVC-LED), UV-transmitting lamps, protective tubing for UV-lamps, UV-transmitting material for UV oxidation reactors, UV flame detectors, UV photoelectric cells, sun reactors, spectral analysis device, photomultiplier, and for windows (in particular EPROM windows), cover plate for solar cells (e.g. in space), UV-transmitting cuvette (e.g. for photoluminescence measurements with UV excitation), UV-CCL (cold cathode lamps) and/or xenon flash lamps.

In one aspect, the invention relates to the use of the glass of the invention in diagnostics, in particular for or as microfluidic component, e.g. for photoluminescence-based diagnostics. The glass may be used for or as the bottom plate or the cover plate of a microfluidic component. The high UV-transmittance is advantageous for the signal-to-noise ratio of the diagnostic method.

In one aspect of the invention, the invention relates to the use of the glass of the invention for lamps emitting UV light, in particular UV lamps with or without protective tubing.

In a particular preferred aspect, the invention relates to the use of a glass of the invention in UVC detectors or as envelope in germicidal lamps.

The invention also relates to a UVC detector or a germicidal lamp comprising the glass of the invention.

## Description of the Figures

Figure 1 shows the emission spectrum of the HOK 4 lamp. On the x-axis the wavelength in nm is shown. On the y-axis the relative intensity in comparison to the maximum intensity is shown.

Figure 2 shows the transmittance spectra of Examples 1, 2 and 3 in a wavelength range of from 200 nm to 400 nm before and after irradiation with the HOK 4 lamp for 144 hours.

## Examples

### 1. Example A

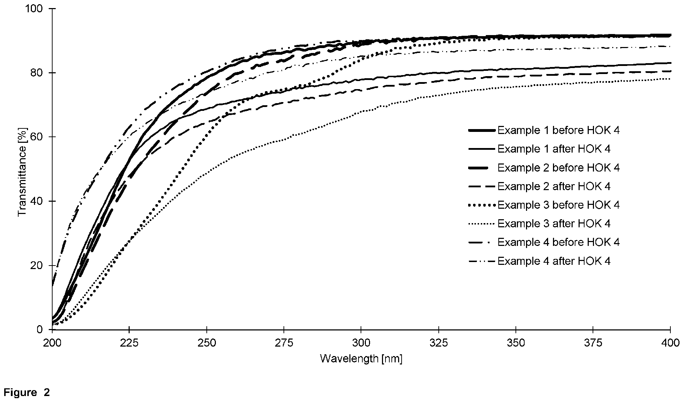

Alkali-containing silicate glasses in accordance with the glass composition as described in the present disclosure have been tested with respect to UV transmittance and solarization resistance. The composition of the glasses merely differed with respect to the amount of $MoO_3$. The amount of $MoO_3$ was 7.4 ppm in example 1, 23 ppm in example 2, 59 ppm in example 3, and less than 3 ppm in example 4. Example 3 is not according to the claimed invention. All examples were obtained under reducing melting conditions by adding sucrose to the glass raw materials in an amount of 0.4 wt.-%. Samples having a thickness of about 0.7 mm have been tested for transmittance in the wavelength range of from 200 nm to 400 nm before and after irradiation with the HOK 4 lamp. The results are shown in Figure 2.

In particular, regarding the particularly relevant wavelength of 260 nm, the results obtained are summarized in the following table.

| | Transmittance at a thickness of 0.7 mm before HOK 4 irradia-tion [%] | Transmittance at a thickness of 0.7 mm af-ter HOK 4 irradiation [%] | Induced Extinction [1/cm] |
|---|---|---|---|
| Example 1 | 82.9 | 71.3 | 2.15 |
| Example 2 | 79.8 | 67.6 | 2.37 |
| Example 3 | 69.9 | 54.4 | 3.58 |
| Example 4 | 84.2 | 77.4 | 1.20 |

The results show that an increasing amount of $MoO_3$ has two different effects. On the one hand, the transmittance is reduced. On the other hand, the induced extinction is increased.

## 2. Example B

Alkali-containing silicate glasses in accordance with the glass composition as described in the present disclosure comprising $MoO_3$ in an amount of about 20 to 25 ppm have been tested with respect to UV transmittance and solarization resistance. The glasses of examples 4 and 5 whose compositions were basically identical (23 ppm $MoO_3$ in example 4 and 21 ppm $MoO_3$ in example 5) mainly differed with respect to the melting conditions applied. Example 4 was obtained under reducing melting conditions by adding sucrose to the glass raw materials. In contrast, example 5 was obtained under oxidizing melting conditions (1 wt.-% nitrate). Example 5 is not according to the claimed invention. Samples having a thickness of about 1.0 mm have been tested for transmittance at a wavelength of 260 nm before and after irradiation with the HOK 4 lamp. The results obtained are summarized in the following table.

| | Transmittance at a thickness of 1.0 mm before HOK 4 irradiation [%] | Transmittance at a thickness of 1.0 mm after HOK 4 irradiation [%] |
|---|---|---|
| Example 4 | 75.8 | 59.9 |
| Example 5 | 64.9 | 53.0 |

The transmittance at a wavelength of 260 nm was higher in example 4 obtained under reducing melting conditions as compared to example 5 obtained under oxidizing melting conditions both before and after irradiation with the HOK 4 lamp.

## Claims

1. Glass, wherein the amount of $MoO_3$ is from 0.1 ppm to 30.0 ppm, and wherein molybdenum is present in such oxidation states that the transmittance at a wavelength of 260 nm is at least 65.0% at a reference thickness of 1.0 mm, wherein the glass comprises the following components in the indicated amounts (in wt.-%):

| Component | Amount (wt.-%) |
|---|---|
| $SiO_2$ | 60.0-80.0 |
| $B_2O_3$ | 0.5-30.0 |
| $Al_2O_3$ | 0.5-10.0 |
| $Na_2O$ | 2.0-20.0 |
| $K_2O$ | 0-20.0 |
| BaO | 0-15.0 |

2. Glass according to claim 1 comprising the following components in the indicated amounts (in wt.-%):

| Component | Amount (wt.-%) |
|---|---|
| $SiO_2$ | 60.0-75.0 |
| $B_2O_3$ | 0.5-5.0 |
| $Al_2O_3$ | 0.5-5.0 |
| $Na_2O$ | 7.0-10.0 |
| $K_2O$ | 8.0-12.0 |
| BaO | 4.5-10.0 |

3. Glass according to at least one of the preceding claims, wherein the glass comprises less than 10 ppm $Fe_2O_3$.

4. Glass according to at least one of the preceding claims, wherein the glass comprises less than 10 ppm $TiO_2$.

5. Glass according to at least one of the preceding claims, wherein the glass comprises less than 10 ppm NiO.

6. Glass according to at least one of the preceding claims, wherein the glass comprises less than 10 ppm $Cr_2O_3$.

7. Glass according to at least one the preceding claims, wherein the glass comprises Cl in an amount of more than 100 ppm.

8. Glass according to at least one of the preceding claim, wherein the proportion of $ZrO_2$ is less than 0.5 wt.-%.

9. Glass according to at least one of the preceding claims, wherein the transmittance at a reference thickness of 1.0 mm at a wavelength of 260 nm after irradiation with the HOK 4 lamp for 144 hours is at least 54.0%.

10. Glass according to at least one of the preceding claims, wherein the induced extinction at a wavelength of 260 nm is at most 3.5/cm, wherein the induced extinction $Ext_{ind}$ is determined based on the transmittance before and after irradiation with a HOK 4 lamp for 144 hours and the thickness of the glass sample using the following formula:

$$Ext_{ind} = -\frac{ln\frac{T_{after}}{T_{before}}}{d}$$

wherein $Ext_{ind}$ is the induced extinction, $T_{after}$ is the transmittance after irradiation with the HOK 4 lamp for 144 hours, $T_{before}$ is the transmittance before irradiation with the HOK 4 lamp for 144 hours, d is the sample thickness, and ln is the natural logarithm.

11. Glass according to at least one of the preceding claims, wherein the molybdenum induced extinction at a wavelength of 260 nm is at most 0.20/(cm*ppm) at a reference amount of $MoO_3$ of 20 ppm, wherein the molybdenum induced extinction $MoExt_{ind}$ is determined based on the transmittance before and after irradiation with the HOK 4 lamp for 144 hours, the thickness of the glass sample, and the amount of $MoO_3$ using the following formula:

$$MoExt_{ind} = -\frac{ln\frac{T_{after}}{T_{before}}}{d \cdot Mo}$$

wherein $MoExt_{ind}$ is the molybdenum induced extinction, $T_{after}$ is the transmittance after irradiation with the HOK 4 lamp for 144 hours, $T_{before}$ is the transmittance before irradiation with the HOK 4 lamp for 144 hours, d is the sample thickness, ln is the natural logarithm, and Mo is the amount of $MoO_3$ in ppm.

12. Glass according to at least one of the preceding claims, wherein the partial pressure of oxygen ($pO_2$) in a glass melt at a temperature of 1500°C is 0.5 bar or less, when the glass melt is produced from the glass by inductively heating to a temperature of 1500°C in a platinum crucible under argon atmosphere, wherein the $pO_2$ of the glass melt may be determined in accordance with the description

13. Method of producing a glass according to at least one of the preceding claims comprising the following steps:

    a) Melting glass raw materials under reducing melting conditions, wherein reducing melting conditions comprises adding one or more reducing agents, for example sugar, to the glass raw materials,
    b) Optionally refining the melt,
    c) Cooling the melt,
    wherein melting step a) comprises use of molybdenum electrodes,
    wherein the method comprises temperatures of 1440 °C or more,
    wherein the method involves viscosities of 2.5 dPas or less.

14. Use of a glass according to at least one of claims 1 to 12 in UVC detectors or as envelope in germicidal lamps.

**Patentansprüche**

1. Glas, wobei die Menge an $MoO_3$ 0,1 ppm bis 30,0 ppm beträgt, und wobei Molybdän in solchen Oxidationszuständen vorhanden ist, dass die Durchlässigkeit bei einer Wellenlänge von 260 nm mindestens 65,0% bei einer Referenzdicke von 1,0 mm beträgt, wobei das Glas die folgenden Komponenten in den angegebenen Mengen (in Gew.-%) umfasst:

| Komponente | Menge (Gew.-%) |
|---|---|
| $SiO_2$ | 60,0-80,0 |
| $B_2O_3$ | 0,5-30,0 |
| $Al_2O_3$ | 0,5-10,0 |
| $Na_2O$ | 2,0-20,0 |
| $K_2O$ | 0-20,0 |
| BaO | 0-15,0 |

2. Glas gemäß Anspruch 1 umfassend die folgenden Komponenten in den angegebenen Mengen (in Gew.-%):

| Komponente | Menge (Gew.-%) |
|---|---|
| $SiO_2$ | 60,0-75,0 |
| $B_2O_3$ | 0,5-5,0 |
| $Al_2O_3$ | 0,5-5,0 |
| $Na_2O$ | 7,0-10,0 |
| $K_2O$ | 8,0-12,0 |
| BaO | 4,5-10,0 |

3. Glas gemäß mindestens einem der vorhergehenden Ansprüche, wobei das Glas weniger als 10 ppm $Fe_2O_3$ umfasst.

4. Glas gemäß mindestens einem der vorhergehenden Ansprüche, wobei das Glas weniger als 10 ppm $TiO_2$ umfasst.

5. Glas gemäß mindestens einem der vorhergehenden Ansprüche, wobei das Glas weniger als 10 ppm NiO umfasst.

6. Glas gemäß mindestens einem der vorhergehenden Ansprüche, wobei das Glas weniger als 10 ppm $Cr_2O_3$ umfasst.

7. Glas gemäß mindestens einem der vorhergehenden Ansprüche, wobei das Glas Cl in einer Menge von mehr als 100 ppm umfasst.

8. Glas gemäß mindestens einem der vorhergehenden Ansprüche, wobei der Anteil an $ZrO_2$ weniger als 0,5 Gew.-% beträgt.

9. Glas gemäß mindestens einem der vorhergehenden Ansprüche, wobei die Durchlässigkeit bei einer Referenzdicke von 1,0 mm bei einer Wellenlänge von 260 nm nach Bestrahlung mit der HOK 4-Lampe 144 Stunden lang mindestens 54,0% beträgt.

10. Glas gemäß mindestens einem der vorhergehenden Ansprüche, wobei die induzierte Extinktion bei einer Wellenlänge von 260 nm höchstens 3,5/cm beträgt, wobei die induzierte Extinktion $Ext_{ind}$ bezogen auf die Durchlässigkeit vor und nach Bestrahlung mit einer HOK 4-Lampe 144 Stunden lang und die Dicke der Glasprobe unter Verwendung der folgenden Formel bestimmt wird:

$$Ext_{ind} = -\frac{ln\frac{T_{nach}}{T_{vor}}}{d}$$

wobei $Ext_{ind}$ die induzierte Extinktion ist, $T_{nach}$ die Durchlässigkeit nach Bestrahlung mit der HOK 4-Lampe 144 Stunden lang ist, $T_{vor}$ die Durchlässigkeit vor Bestrahlung mit der HOK 4-Lampe 144 Stunden lang ist, d die Probendicke ist, und In der natürliche Logarithmus ist.

11. Glas gemäß mindestens einem der vorhergehenden Ansprüche, wobei die Molybdän-induzierte Extinktion bei einer Wellenlänge von 260 nm höchstens 0,20/(cm*ppm) bei einer Referenzmenge an $MoO_3$ von 20 ppm beträgt, wobei die Molybdän-induzierte Extinktion $MoExt_{ind}$ bezogen auf die Durchlässigkeit vor und nach Bestrahlung mit der HOK 4-Lampe 144 Stunden lang, die Dicke der Glasprobe, und die Menge an $MoO_3$ unter Verwendung der folgenden Formel bestimmt wird:

$$MoExt_{ind} = -\frac{ln\frac{T_{nach}}{T_{vor}}}{d \cdot Mo}$$

wobei $MoExt_{ind}$ die Molybdän-induzierte Extinktion ist, $T_{nach}$ die Durchlässigkeit nach Bestrahlung mit der HOK 4-Lampe 144 Stunden lang ist, $T_{vor}$ die Durchlässigkeit vor Bestrahlung mit der HOK 4-Lampe 144 Stunden lang ist, d die Probendicke ist, In der natürliche Logarithmus ist, und Mo die Menge an $MoO_3$ in ppm ist.

12. Glas gemäß mindestens einem der vorhergehenden Ansprüche, wobei der Partialdruck von Sauerstoff ($pO_2$) in einer Glasschmelze bei einer Temperatur von 1500°C 0,5 bar oder weniger beträgt, wenn die Glasschmelze aus dem Glas durch induktives Erwärmen auf eine Temperatur von 1500°C in einem Platintiegel unter Argonatmosphäre hergestellt wird, wobei der $pO_2$ der Glasschmelze gemäß der Beschreibung bestimmt werden kann.

13. Verfahren zur Herstellung eines Glases gemäß mindestens einem der vorhergehenden Ansprüche umfassend die folgenden Schritte:

a) Schmelzen von Glasrohmaterialien unter reduzierenden Schmelzbedingungen, wobei reduzierende Schmelzbedingungen Zugeben von einem oder mehr reduzierenden Mitteln, zum Beispiel Zucker, zu den Glasrohmaterialien umfassen,
b) gegebenenfalls Läutern der Schmelze,
c) Kühlen der Schmelze,

wobei Schmelzschritt a) Verwendung von Molybdänelektroden umfasst,
wobei das Verfahren Temperaturen von 1440°C oder höher umfasst,
wobei das Verfahren Viskositäten von 2,5 dPas oder niedriger einbezieht.

14. Verwendung eines Glases gemäß mindestens einem der Ansprüche 1 bis 12 in UVC-Detektoren oder als Umhüllung in Entkeimungslampen.

**Revendications**

1. Verre, dans lequel la quantité de $MoO_3$ est comprise entre 0,1 ppm et 30,0 ppm, et dans lequel le molybdène est présent dans des états d'oxydation tels que le facteur de transmission à une longueur d'onde de 260 nm est d'au moins 65,0 % à une épaisseur de référence de 1,0 mm, dans lequel le verre comprend les composants suivants dans les quantités indiquées (en % en poids) :

| Composant | Quantités (% en poids) |
|---|---|
| $SiO_2$ | 60,0-80,0 |
| $B_2O_3$ | 0,5-30,0 |
| $Al_2O_3$ | 0,5-10,0 |
| $Na_2O$ | 2,0-20,0 |
| $K_2O$ | 0-20,0 |
| BaO | 0-15,0 |

2. Verre selon la revendication 1, comprenant les composants suivants dans les quantités indiquées (en % en poids) :

| Composant | Quantités (% en poids) |
|---|---|
| $SiO_2$ | 60,0-75,0 |
| $B_2O_3$ | 0,5-5,0 |
| $Al_2O_3$ | 0,5-5,0 |
| $Na_2O$ | 7,0-10,0 |
| $K_2O$ | 8.0-20,0 |
| BaO | 4.5-10,0 |

3. Verre selon au moins l'une des revendications précédentes, dans lequel le verre comprend moins de 10 ppm de $Fe_2O_3$.

4. Verre selon au moins l'une des revendications précédentes, dans lequel le verre comprend moins de 10 ppm de $TiO_2$.

5. Verre selon au moins l'une des revendications précédentes, dans lequel le verre comprend moins de 10 ppm de NiO.

6. Verre selon au moins l'une des revendications précédentes, dans lequel le verre comprend moins de 10 ppm de $Cr_2O_3$.

7. Verre selon au moins l'une des revendications précédentes, dans lequel le verre comprend du CI en une quantité supérieure à 100 ppm.

8. Verre selon au moins l'une des revendications précédentes, dans lequel la proportion de $ZrO_2$ est inférieure à 0,5 % en poids.

9. Verre selon au moins l'une des revendications précédentes, dans lequel le facteur de transmission à une épaisseur de référence de 1,0 mm à une longueur d'onde de 260 nm après irradiation avec la lampe HOK 4 pendant 144 heures est d'au moins 54,0 %.

10. Verre selon au moins l'une des revendications précédentes, dans lequel l'extinction induite à une longueur d'onde de 260 nm est d'au plus 3,5/cm, dans lequel l'extinction induite $Ext_{ind}$ est déterminée sur la base du facteur de transmission avant et après irradiation avec une lampe HOK 4 pendant 144 heures et de l'épaisseur de l'échantillon de verre en utilisant la formule suivante :

$$Ext_{ind} = -\frac{ln\frac{T_{after}}{T_{before}}}{d}$$

où $Ext_{ind}$ est l'extinction induite, $T_{after}$ est le facteur de transmission après irradiation avec la lampe HOK 4 pendant 144 heures, $T_{before}$ est le facteur de transmission avant irradiation avec la lampe HOK 4 pendant 144 heures, d est l'épaisseur de l'échantillon, et In est le logarithme népérien.

11. Verre selon au moins l'une des revendications précédentes, dans lequel l'extinction induite par le molybdène à une longueur d'onde de 260 nm est d'au plus 0,20/(cm*ppm) pour une quantité de référence de $MoO_3$ de 20 ppm, l'extinction induite par le molybdène $MoExt_{ind}$ est déterminée sur la base du facteur de transmission avant et après irradiation avec la lampe HOK 4 pendant 144 heures, de l'épaisseur de l'échantillon de verre et de la quantité de $MoO_3$ en utilisant la formule suivante :

$$MoExt_{ind} = -\frac{ln\frac{T_{after}}{T_{before}}}{d \cdot Mo}$$

où $MoExt_{ind}$ est l'extinction induite par le molybdène, $T_{after}$ est le facteur de transmission après irradiation avec la lampe HOK 4 pendant 144 heures, $T_{before}$ est le facteur de transmission avant irradiation avec la lampe HOK 4 pendant 144 heures, d est l'épaisseur de l'échantillon, In est le logarithme népérien, et Mo est la quantité de $MoO_3$ en ppm.

12. Verre selon au moins l'une des revendications précédentes, dans lequel la pression partielle d'oxygène ($pO_2$) dans une masse fondue de verre à une température de 1500°C est de 0,5 bar ou moins, lorsque la masse fondue de verre est produite à partir du verre par chauffage par induction à une température de 1500°C dans un creuset en platine sous atmosphère d'argon, dans lequel la $pO_2$ de la masse fondue de verre peut être déterminée conformément à la description.

13. Procédé de production d'un verre selon au moins l'une des revendications précédentes comprenant les étapes suivantes :

   a) fusion de matières premières de verre dans des conditions de fusion réductrices, dans laquelle les conditions de fusion réductrices comprennent l'ajout d'un ou plusieurs agents réducteurs, par exemple du sucre, aux matières premières de verre,
   b) affinage facultatif de la masse fondue,
   c) refroidissement de la masse fondue,

   dans lequel l'étape de fusion a) comprend l'utilisation d'électrodes en molybdène,
   dans lequel le procédé comprend des températures de 1440°C ou plus, dans lequel le procédé implique des viscosités de 2,5 dPas ou moins.

14. Utilisation d'un verre selon au moins l'une des revendications 1 à 12 dans des détecteurs UVC ou comme enveloppe dans des lampes germicides.

Figure 1

Figure 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020121114 A1
- EP 1101740 A1